Europäisches Patentamt

(19) European Patent Office

Office européen des brevets

(11) EP 0 792 865 A1

(12) EUROPEAN PATENT APPLICATION

(43) Date of publication:
03.09.1997 Bulletin 1997/36

(51) Int. Cl.$^6$: C07C 51/235

(21) Application number: 97103092.9

(22) Date of filing: 26.02.1997

(84) Designated Contracting States:
DE ES FR IT

(30) Priority: 27.02.1996 US 607728

(71) Applicant: PRAXAIR TECHNOLOGY, INC.
Danbury, CT 06810-5113 (US)

(72) Inventors:
• Kingsley, Jeffrey Paul
East Amherst, New York 14051 (US)

• Litz, Lawrence Marvin
Pleasantville, New York 10570 (US)
• Iacopelli, Frank Salvatore
Yonkers, New York 10703 (US)
• Weise, Mark Kurt
Wayne, New Jersey 07470 (US)

(74) Representative: Schwan, Gerhard, Dipl.-Ing.
Elfenstrasse 32
81739 München (DE)

(54) Process for producing organic acids

(57) Oxo acids are produced by the controlled oxidation of aldehydes using oxygen rather than feed air in a gas-liquid contacting system that eliminates oxygen-deficient zones within the reaction volume and minimizes the amount of oxygen passing to the headspace in the system.

**Description**

BACKGROUND OF THE INVENTION

Field of The Invention

The invention relates to the production of organic acids. More particularly, it relates to the production of Oxo acids.

Background of the Invention

Aliphatic acids are provided by liquid phase reaction of an aldehyde with oxygen according to the reaction:

$$R\text{-}HO + 1/2 O_2 = R\text{-}OOH$$

The aldehydes, and corresponding acids, may be linear or branched, and the number of carbon atoms may vary from 3 to 12. The precursor aldehydes are often made using the Low Pressure Oxo (LPO) process. Hence, the derivative acids are often referred to as Oxo acids. The aldehydes may also be obtained or produced by means other than the LPO process but this class of compounds is often referred to as Oxo acids none the less. The source of the aldehydes is not critical to this invention.

In commercial Oxo acid production, selectivity to acid is typically between 80% and 99%. Selectivity decreases with chain length and the number of side chains or branches. For example, the selectivity of propionaldehyde which has three carbon atoms (C3), to propionic acid, is better than the selectivity of valeraldehyde to valeric acid, which has 5 carbon atoms (C5); and the selectivity of valeraldehyde, which is a linear five carbon molecule, to valeric acid is higher than the selectivity of 2-methyl butyraldehyde, which is a branched C5, to 2-methyl butyric acid. By-product inhibitor additives may be added to some of these systems to improve selectivity.

In liquid phase aldehyde oxidation, the oxygen is typically introduced into the liquid by mass transfer from gaseous air bubbles. The oxidation reactions occur in the liquid phase, either in the bulk liquid phase or in the liquid film which surrounds the air bubbles. Oxygen starvation, that is lack of dissolved oxygen in the reaction liquid, promotes by-product formation reactions and hence reduces the selectivity of aldehyde to acid. Thus, adequate mass transfer of oxygen from the gas phase to the liquid phase is critical to maintain adequate dissolved oxygen concentration in the liquid phase in order to suppress by-product formation reactions.

By-product formation increases, not surprisingly, with reaction temperature. Since reaction rate typically increases with temperature, the reaction consumes oxygen faster at higher temperature, and more oxygen is required to prevent the onset of oxygen starved conditions. Thus, gas-liquid mass transfer limitations become worse as temperature is increased, and therefore, it is more difficult to prevent oxygen starved conditions which cause by-product formation. The by-products formed under oxygen starved conditions are formate esters, ketones and alcohols.

Since the conversion of aldehyde to acid increases with temperature, it is possible to increase reactor productivity by increasing temperature. However, if the increase in temperature moves the reaction system into the oxygen starved regime, or makes an already oxygen starved condition worse, by-product formation reactions increase and selectivity to acid will decrease.

Oxo acids are typically produced in air sparged stirred tank or gas lift bubble column reactors. At commercial reaction conditions, the exothermic heat of reaction produced by the oxidation reactions is significant. Gas lift bubble column reactors are preferred because they can be packed with heat transfer tubes to give a higher heat transfer surface area to volume ratio than can be achieved in practical configurations of conventional stirred tank reactors with cooling coils. Stirred tanks have been used in commercial operations, but volumetric productivity is lower than bubble columns due to limited heat transfer capacity. Feed air is usually introduced into these reactors through gas spargers which produce a dispersion of gas bubbles.

Air based bubble columns and stirred tank reactor systems have inherent mass transfer limitations due to the low concentration of oxygen in air. Oxygen mass transfer is proportional to the oxygen concentration or oxygen partial pressure in the gas bubble. The concentration of oxygen in an air bubble in a bubble column or a stirred tank reactor is 21% at the sparger. The oxygen concentration decreases well below 21% as oxygen dissolves into the reaction liquid where it is consumed by the reaction, and as liquid evaporates into the air bubble. The oxygen partial pressure in the air bubbles decreases, while the partial pressure of nitrogen which is a component of air, and the partial pressure of evaporated organic material increase.

In conventional stirred tank and bubble column reactor designs, the oxygen partial pressure of the exiting waste air stream must be maintained below a practical safety limit of 5% in order to prevent formation of flammable gas mixtures in the reactor vapor space. Thus gas phase oxygen concentration in conventional reactor designs is constrained between 21% at the air feed point and 5% at the waste gas exit. In bubble columns, the air is injected at the bottom of the reactor. The oxygen concentration on an organic free basis varies from 21% at the bottom to 5% at the top of the reactor. In a well mixed stirred tank reactor, the average oxygen concentration in the system is 5% throughout. Thus for a given operating pressure, safety concerns in conventional reactor systems severely constrain the available mass transfer driving force. The situation can be improved somewhat by raising the overall system pressure, which raises the oxygen partial pressure, or by purging the headspace with relatively large amounts of an inert gas such as nitrogen, but these alternatives are generally very expensive.

Another factor which limits oxygen mass transfer capacity is the degree to which the oxygen containing gas bubbles are uniformly distributed within the liquid phase. If some regions of the liquid phase are not exposed to oxygen containing gas bubbles, those regions will be oxygen starved and by-product formation will occur. Hence, it is crucial to have good gas bubble distribution throughout the reactor.

In conventional bubble column reactors, or gas lift bubble column reactors, the gas bubbles are introduced at the bottom of the reactor. They rise through the reaction liquid due to their buoyancy. The bubbles cause a recirculating liquid flow pattern. In bubble column reactors, the flow tends to be up through the center of the reactor and down near the walls of the reactor. The oxygen containing gas bubbles tend to concentrate in the center upflow region, which leaves the outer downflow region gas starved and subject to by-product formation reactions. In gas lift bubble columns, the oxygen is typically sparged into one or more tubes such that the gas causes liquid upflow in the tubes. Additional tubes without spargers are provided for recirculating flow. Oxygen starved conditions, and hence by-product formation reactions, prevail in the downflow tubes.

Gas distribution problems are not as prevalent in well mixed stirred tank reactors, since uniform gas dispersion is easier to accomplish in a well mixed system.

The net result of the limited mass transfer driving force that is inherent in conventional air based reactor systems is that oxygen starved conditions, and the accompanying selectivity penalty, are more likely to occur as reaction temperature and reactor productivity are increased.

It is an object of the invention, therefore, to provide an improved process for the production of Oxo acids.

It is another object of the invention to provide a process for the production of Oxo acids with improved selectivity and productivity.

With these and other objects in mind, the invention is hereinafter described in detail, the novel features thereof being particularly pointed out in the appended claims.

Summary of the Invention

Oxo acids are produced by controlled oxidation of aldehydes using oxygen instead of feed air, together with a gas-liquid contacting system that eliminates oxygen deficient zones within the reaction volume.

Detailed Description of the Invention

The objects of the invention are accomplished by the use of pure or nearly pure oxygen as the feed gas in the place of feed air and by the carrying out of the oxidation reaction in an LOR reaction system in place of conventional reaction systems, as described above, for the oxidation of Oxo aldehydes to produce the desired Oxo acids.

In the oxidation of organic liquids, it is necessary to prevent a potentially explosive or flammable vapor-gas phase mixture from developing in the overhead gas phase from exceeding the lower flammability limit for a particular operation. A Liquid Organic Reactor (LOR) process and system, as described and claimed in the Litz et al. patent, U.S. No. 4,900,480, has been found to be advantageous for such organic liquid oxidation applications.

By replacing air with oxygen, the partial pressure of oxygen containing gas bubbles within the oxidation reactor is significantly greater than the inherently limited oxygen partial pressure in air. Consequently, the driving force for mass transfer is greater, and the likelihood of oxygen starved conditions, which cause by-product formation is lower, such oxygen used as the feed gas.

The LOR system is a well mixed stirred reactor system, with oxygen bubbles uniformly distributed throughout the reactant liquid. The LOR approach, in its most common embodiment, uses a mixing impeller and draft tube arranged to disperse and circulate gas bubbles in the liquid phase. When used to safely react gaseous oxygen with flammable liquids, the gas bubbles comprise a mixture of feed oxygen, flammable organic vapor and by-product gases. With the gas bubbles dispersed as small bubbles throughout the liquid phase, the flammability hazard associated with the oxygen and the organic vapor mixture is mitigated by the heat capacity of the surrounding liquid, which adsorbs the heat or reaction in the event of bubble ignition, and because the flame from a single bubble cannot propagate through the liquid phase.

In the LOR system as described in the Litz patent, a recirculating liquid reaction zone is separated from, but remains in fluid communication with, a quiescent zone that is in contact with the overhead gas phase, typically as shown in Fig. 2 of the patent. A baffle between said zones serves to substantially prevent gas bubbles that are carried with the liquid in the recirculating liquid zone from disengaging the liquid because of their buoyancy, thus insuring that the bubbles are recirculated with the liquid and are efficiently consumed by reaction. Any gas bubbles that do escape from the recirculating liquid zone under the baffle, and pass upward through the quiescent zone, are collected in the gas space above the quiescent zone, where they are rendered non-flammable by the addition of inert gas, e.g. nitrogen, to said gas space.

Since the LOR system is a well mixed stirred tank reaction system, oxygen bubbles are generally uniformly distributed throughout the liquid. Thus, in the operation of the LOR system in the practice of the invention, there are essentially no zones in the reactor that are oxygen starved due to poor gas-liquid contacting. In addition, the requirement that the gas bubbles have a concentration of 5% or less does not apply when the LOR system is employed, as it does in conventional reactor systems. Consequently, depending on the vapor pressure of the liquid, which acts as a diluent, the average oxygen concentration in the gas bubble is much higher in an LOR system than it is in a conventional reactor. In LOR systems with a very low liquid vapor pressure, the average oxygen concentration can approach 95% or higher. This compares favorably to the average 5% oxygen concentration in a conventional air based stirred tank reactor and to the average of about 13% in an air based bubble column reactor.

For aldehyde oxidation in Oxo acid production, the higher overall mass transfer rate associated with oxygen based LOR technology increases the amount of oxygen available for reaction in the liquid phase and thereby reduces selectivity losses that are associated with oxygen starved conditions.

Furthermore, for a given aldehyde conversion rate, the overall higher mass transfer rate that is obtained with oxygen allows for operation at lower temperature and pressure than in conventional air based reaction systems. Since the gas phase oxygen concentration is much higher in an oxygen based system, the same oxygen partial pressure can be achieved at a much lower total pressure than with air. Also, since both temperature and oxygen concentration increase the reaction rate, a given reaction rate can be maintained by increasing the oxygen concentration and lowering the reaction temperature. Operation at such lower temperature further reduces by-product formation and increases product selectivity.

An important advantage of the LOR system is that it is a well mixed stirred tank reactor system having separate reaction and gas disengagement zones that are defined by the baffle means as described with reference to the Figs. 1 and 2 embodiments of Litz et al., or the corresponding means of the Figs. 3 and 4 embodiments thereof. Since said baffle or other means keeps most of the unreacted oxygen from disengaging from the liquid before it is reacted, very little inert gas is required to assure that the headspace gas is below the flammable limit, which, with acceptable safety margins, is typically 5% oxygen or less.

Those skilled in the art will appreciate that various changes and modifications can be made in the details of the process as herein described without departing from the scope of the invention as set forth in the appended claims. As indicated above, it is convenient and generally desirable to use the LOR system in the Fig. 2 embodiment thereof, but that the other embodiments thereof can also be employed in the practice of the invention. Furthermore, while essentially pure oxygen, e.g. 99% pure oxygen, can be employed as the oxygen containing gas used for the oxidation of aldehydes, lower purity oxygen can also be used for such purpose. Thus, oxygen containing gas having an oxygen concentration of about 50% by volume or above will offer improved selectivity over the use of feed air. The magnitude of the improved selectivity will generally increase in proportion to the oxygen concentration in the oxygen containing gas. In particular, oxygen of greater than 90% purity should generally result in nearly the same benefit as is obtained using 99% pure oxygen.

While oxygen may be used to advantage in a bubble column or gas lift bubble column reactors to improve performance relative to feed air in these reactor configurations due to the higher available oxygen partial pressure, a large quantity of inert gas is necessary to inert the headspace in these reactors, rendering the use of such reactors less desirable than the practice of the invention as herein described and claimed. The benefits of oxygen can also be realized in other well mixed stirred tank reactor configurations. However, as in the bubble column approach, the amount of unreacted oxygen that escapes into the reactor headspace will be greater in non-LOR stirred tank systems. Hence, the amount of inert gas required to maintain nonflammable conditions in the headspace is typically much greater than for LOR operations. Processes using such non-LOR stirred tank systems are again economically unfavorable compared to the use of an LOR approach because of the high cost of nitrogen or other inert purge gas and the higher costs associated with the removal of organic compounds from the purge gas prior to the discharge of said purge gas to the atmosphere.

As indicated above, the feed aldehydes, and the corresponding Oxo acids produced in the practice of the invention, may be linear or branched, and the number of carbon atoms (in R of the indicated reaction) may vary from 3 to 12. While the illustrative example below is based on the oxidation of 2-ethylhexaldehyde to 2-ethylhexanoic acid, any other applicable oxidation of an aldehyde to an aliphatic acid, i.e. an Oxo acid, such as described in the background description above, can be carried out in accordance with the subject invention.

In illustrative examples of the practice of the invention, 2-ethylhexaldehyde was oxidized to 2-ethylhexanoic acid using greater than 99% pure oxygen, as in preferred practice, in a one gallon LOR reaction vessel based on the Fig. 2 embodiment of Litz et al. Improved selectivity was achieved as compared to commercial scale air based reactors. A by-product inhibitor was added to the feed aldehyde. The ratio of feed aldehyde to inhibitor was consistent with conventional practice in the oxidation of aldehydes to produce Oxo acids.

The results of such illustrative examples of the invention as compared to an air based, commercial bubble column reactor are shown in the following Table:

TABLE

| SYSTEM | TEMP (F) | PRESSURE (psig) | MAXIMUM REACTION RATE (mmol/l /min) | ALDEHYDE CONVER- SION | SELECTIVITY | OXYGEN EFFI- CIENCY |
|---|---|---|---|---|---|---|
| 02LOR | 115 | 2 | 55 | 90.6% | 96.6% | 92.9% |
| 02LOR | 117 | 2.5 | 62 | 96.6% | 96.6% | 84.5% |
| 02LOR | 115 | 3.5 | 54 | 90.2% | 94.7% | 99.1% |
| 02LOR | 95 | 22 | 186 | 99.4% | 95.0% | 99.9% |
| 02LOR | 110 | 30 | 172 | 99.9% | 96.3% | 100.0% |
| 02LOR | 110 | 30 | 177 | 99.7% | 96.1% | 100.0% |
| 02LOR | 118 | 32 | 111 | 96.4% | 96.7% | 100.0% |
| AIR PLANT | 140-212 | 100-120 | 104 | 92.0% | 92.0% | - |

The results in the Table clearly show that improved selectivity and higher overall reaction rates can be achieved using oxygen feed and an LOR reactor system as compared to conventional air based reactor systems. Furthermore, improved performance was achieved at much lower temperature and pressure by the use of an oxygen based LOR system than by a conventional air based reactor system.

Depending on the particular oxidation reaction being carried out in any application of the invention, the reaction temperature will generally range from about 90°F to about 160°F. The operating pressure will generally range from about 15 to 45 psig, and will typically be on the order of about 40 psig. For any particular aldehyde oxidation reaction being carried out, the operating temperature and pressure will generally be significantly lower than that employed in conventional practice, while achieving improved performance as illustrated by the results of the illustrative examples shown in the Table.

The production of Oxo acids is a commercially significant operation. The invention enhances the feasibility of carrying out such production activities in a highly desirable manner satisfying the need in the act for improved performance in an efficient, economically favorable oxidation reaction.

## Claims

1.   An improved process for the production of aliphatic acids by the oxidation of aliphatic aldehydes comprising:

(a) maintaining a portion of a body of liquid containing said aliphatic aldehydes in a recirculating flow condition in a reactor vessel, said recirculating portion of the body of liquid having no gas-liquid interface with an overhead gas phase, said recirculating portion of the body of liquid being separated from, but in fluid communication with, a relatively quiescent portion of the body of liquid, said quiescent portion of the body of liquid having a gas-liquid interface with a headspace gas phase and being adapted to accommodate a change in liquid level in response to a change in volume in the body of liquid between the condition in which essentially no gas bubbles are in the body of liquid and the condition that exists when a desired gas bubble concentration is developed within said body of liquid;
(b) introducing a feed gas stream of essentially pure oxygen, or oxygen-enriched air containing about 50% or more oxygen, directly into the recirculating portion of the body of liquid, and not into the quiescent portion thereof, the recirculating flow path and flow velocity of said recirculating portion of the body of liquid being such, relative to the fluid communication between said recirculating and quiescent portions of the body of liquid, that the oxygen bubbles formed upon introduction of the feed oxygen into the recirculating portion of the body of liquid are maintained in dispersed form in the recirculating liquid, without oxygen deficient zones, for oxygen dissolution in, and reaction with, the aliphatic aldehydes in the recirculating portion of the body of liquid, without appreciable passage of oxygen bubbles through the fluid communication between the recirculating portion of the body of liquid and the quiescent portion thereof to the gas-liquid interface, and thus without any appreciable loss of oxygen to the overhead gas phase;

(c) maintaining the oxygen-aliphatic aldehyde liquid mixture in the reaction vessel at a temperature of from about 90°F to about 160°F, and a pressure of between about 15 psig and about 45 psig; and

(d) recovering desired aliphatic acid product from the reactor vessel,

whereby said aliphatic acid can be efficiently produced without the presence of oxygen deficient zones within the recirculating portion of the body of liquid, and with enhanced selectivity and reaction rates for the production of the desired aliphatic acid product and minimized production of undesired by-products.

2. The process of Claim 1 in which the feed gas stream comprises essentially pure oxygen.

3. The process of Claim 1 in which said feed stream comprises oxygen-enriched air containing at least about 50% oxygen.

4. The process of Claim 1 in which said recirculating portion of the body of liquid is maintained in a recirculating flow condition by the essentially central positioning therein of a hollow draft tube such that open ends thereof are at the bottom and top thereof, the oxygen-aliphatic aldehyde liquid mixture in the recirculating portion of the body of liquid being caused to pass through the hollow draft tube by impeller means positioned therein.

5. The process of Claim 1 in which said aliphatic aldehydes comprise aldehydes having from 3 to 12 carbon atoms.

6. The process of Claim 1 in which said aliphatic aldehyde comprises 2-ethylhexaldehyde.

7. The process of Claim 1 in which said aliphatic aldehyde comprises propionaldehyde.

8. The process of Claim 1 in which said aliphatic aldehyde comprises valeraldehyde.

9. The process of Claim 1 in which said aliphatic aldehyde comprises 2-methyl butyric acid.

10. The process of Claim 4 in which said aliphatic aldehydes comprise aldehydes having from 3 to 12 carbon atoms.

11. The process of Claim 10 in which said aliphatic aldehyde comprises 2-ethylhexaldehyde.

12. The process of Claim 11 in which the reaction temperature is about 95°F to about 120°F.

**European Patent Office**

## EUROPEAN SEARCH REPORT

Application Number

EP 97 10 3092

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | EP 0 264 905 A (UNION CARBIDE CORPORATION)<br>* column 1, line 51 - line 55 *<br>* column 2, line 33 - column 13, line 57 *<br>* claims 1-5,11,19-25,30-35 *<br>--- | 1-7 | C07C51/235 |
| Y | EP 0 682 000 A (PRAXAIR TECHNOLOGY, INC.)<br>ENTIRE DOCUMENT.<br>--- | 1 | |
| Y | EP 0 682 005 A (PRAXAIR TECHNOLOGY, INC.)<br>ENTIRE DOCUMENT.<br>----- | 1 | |

| | |
|---|---|
| | **TECHNICAL FIELDS SEARCHED** (Int.Cl.6) |
| | C07C |

The present search report has been drawn up for all claims

| Place of search | Date of completion of the search | Examiner |
|---|---|---|
| THE HAGUE | 3 June 1997 | Klag, M |

EPO FORM 1503 03.82 (P04C01)